# EUROPEAN PATENT SPECIFICATION

(11) **EP 3 115 046 B1**
(45) Date of publication and mention of the grant of the patent: **24.03.2021**
(21) Application number: 16173377.9
(22) Date of filing: 29.09.2009
(51) Int. Cl.: A23K 20/10, A61K 31/19, A61K 31/216, A61K 36/74, A23K 50/40, A23K 20/111

(54) **USE OF AN ANTIOXIDANT COMPONENT IN A PET FOOD COMPOSITION**
VERWENDUNG EINER ANTIOXIDANSKOMPONENTE IN EINER HAUSTIERFUTTERZUSAMMENSETZUNG
UTILISATION D'UN COMPOSANT ANTIOXYDANT DANS UNE COMPOSITION ALIMENTAIRE POUR ANIMAUX DE COMPAGNIE

(30) Priority: 30.10.2008 US 261468
(43) Date of publication of application: 11.01.2017
(62) Divisional of application: 09736731.2
(73) Proprietor: Tetra GmbH, 49324 Melle (DE)
(72) Inventor: WARD, Susan Ruth, Oregonia, OH 45054 (US); TAYLOR, Matthew Joel, Cincinnati, OH 45251 (US)
(74) Representative: Zimmermann & Partner Patentanwälte mbB

(56) References cited:
- EP-A1- 0 299 106
- WO-A-02/071874
- WO-A-2005/074719
- WO-A1-02/085397
- US-A1- 2007 286 925
- J W Hilton ET AL: "Antioxidants: function, types and necessity of inclusion in pet foods", The Canadian veterinary journal = La revue vétérinaire canadienne, 1 August 1989 (1989-08-01), pages 682-684, XP055320039, Canada Retrieved from the Internet: URL:https://www.ncbi.nlm.nih.gov/pmc/artic les/PMC1681164/pdf/canvetj00561-0072.pdf
- IWAI K ET AL: "In Vivo antioxidative effects and tyrosinase inhibitory activities of seven hydroxycinnamoyl derivatives in green coffee beans", JOURNAL OF AGRICULTURAL AND FOOD CHEMISTRY, AMERICAN CHEMICAL SOCIETY, US, vol. 52, 28 July 2004 (2004-07-28), pages 4893-4898, XP003006730, ISSN: 0021-8561, DOI: 10.1021/JF040048M
- R. C. Alves ET AL: "Determination of Vitamin E in Coffee Beans by HPLC Using a Micro-extraction Method", , 22 May 2009 (2009-05-22), pages 1-3, XP055529068, Retrieved from the Internet: URL:https://journals.sagepub.com/doi/10.11 77/1082013208102695 [retrieved on 2018-11-30]

## Description

### FIELD

Described herein but not part of the present invention is a pet food composition comprising an antioxidant component comprising polyphenolic antioxidants recovered from a decaffeination stream produced during the decaffeination process of coffee beans.

The present invention relates to the use of an antioxidant component as defined in claim 1.

### BACKGROUND

Lipid oxidation is a source of deterioration in foods. Lipid oxidation can cause negative affects on the odor, flavor, texture, color and nutritional value of the food. Many food products contain animal tissue of high lipid content or added fat in order to improve product texture. The higher the fat content in the food, the greater the concern that the lipids in the food will oxidize. Food with low fat content, but containing highly oxidizable fats, such as fish oils, also are of great concern regarding oxidation. Oxidative rancidity leads to qualitative and quantitative deterioration of the animal tissues, fats, and oils, resulting in unpalatable odor and flavor, thereby resulting in a shortened shelf life of the food product. One manner of preventing or decreasing lipid oxidation is to add an antioxidant to the food product.

Antioxidants (AOXs) are substances that provide an additional benefit of protecting cells from the damages caused by unstable molecules, such as free radicals and active oxygen species. Such damage may lead to cancer, and thus protecting cells via antioxidants has become a leading interest in the fight against cancer as studies now indicate that antioxidants may slow or possibly prevent the development of cancer.

Oxidation or oxidative stress may be a primary cause of many chronic diseases, including cancer, as well as the aging process itself. As a result, much research exists and is ongoing related to the role that antioxidants play in hindering oxidation, thereby delaying or preventing oxidative stress. Both endogenous and exogenous antioxidants may play a role in controlling oxidation and preventing disease.

In practice, antioxidants interact with and stabilize free radicals and may prevent some of the damage free radicals otherwise might cause. Antioxidants neutralize free radicals as the natural by-product of normal cell processes. Antioxidants are often described as "mopping up" free radicals, meaning they neutralize the free radical and prevent the free radical from taking electrons from other molecules.

One type of antioxidant is the group of compounds called polyphenols. Polyphenols are common constituents of foods of plant origin and contribute the major antioxidants found in diets. The main dietary sources of polyphenols are fruits, vegetables, and beverages. For example, a typical cup of coffee may contain 70-350 mg chlorogenic acids (CGAs), the predominant polyphenolic compound in coffee.

Several thousand different polyphenols have been identified in foods. The two main types of polyphenols are flavonoids and phenolic acids. Some of the more common flavanoids are quercetin (found in onion, tea, apple), catechin (tea, fruit), hesperidin (citrus fruits), and cyanidin (red fruits). One of the most common phenolic acids is caffeic acid, present in many fruits and vegetables. Caffeic acid, most often esterified with quinic acid as in chlorogenic acid, is the major phenolic compound in coffee.

As antioxidants, these polyphenols may protect cell constituents against oxidative damage and therefore reduce the risk of various degenerative diseases, such as cancer, cardiovascular disease, neurodegenerative diseases, diabetes, etc. These degenerative diseases are associated with oxidative damage to cell components, DNA, proteins, and lipids. Antioxidants present in foods and beverages can help limit this damage by acting directly on reactive oxygen species or by stimulating endogenous cell defense systems. The phenolic group in polyphenols can donate hydrogen to a radical, thereby disrupting chain oxidation reactions in cellular components. Epidemiological studies have clearly shown that diets rich in plant foods protect humans against degenerative diseases, such as cancer and cardiovascular disease. As mentioned, plant foods contain a variety of polyphenolic compounds, which are increasingly shown to be effective protective agents (See: Manach et al., 2005, Am. J. Clin. Nutri. 81: 230S-42S).

Increasingly, scientific research is discovering that coffee has a surprisingly high number of beneficial health effects, including as a source of antioxidants. The results of epidemiological studies suggest that coffee consumption is associated with decreased risk of type 2 diabetes, Parkinson's disease, and liver disease. For example, men who drank at least 6 cups of coffee daily had a risk of developing type 2 diabetes that was 54% lower than men who did not drink coffee, and women who drank at least 6 cups of coffee daily had a risk of type 2 diabetes that was 29% lower than women who did not drink coffee (See: Salazar-Martinez et al., 2004, Ann Intern Med. 140: 1-8). In a prospective study of 47,000 men, those who regularly consumed at least one cup of coffee daily had a 40% lower risk of developing Parkinson's disease over the next 10 years than men who did not drink coffee (See: Ascherio et al., 2001, Ann Neurol. 50: 56-63). Evidence also suggests that drinking coffee reduces the risk of colon cancer, cirrhosis, and liver cancer.

As mentioned above and as is well known in the scientific and medical arts, coffee contains a high level of chlorogenic acids, about 5% on a dry weight basis. Chlorogenic acids are polyphenolic antioxidants, similar to the healthy polyphenols that are present in other natural products such as tea, berries, and vegetables. Thus, these chlorogenic acid coffee antioxidants, as well as others, provide health benefits through direct action against free radicals, as mentioned above, as well as indirect actions by modifying metabolism including activation of cellular defenses. Given the increasing emphasis on health and well-being in society, these benefits are important and have become increasingly recognized. Coffee AOXs are also effective as preservatives for food and beverages, decreasing flavor deterioration and fat oxidation, like more traditional AOXs, such as BHA (butylated hydroxyanisole, a synthetic antioxidant) or Vitamin E. Other known benefits include use of chlorogenic acids and polyphenols as flavor pre-cursors.

Moreover, growing knowledge about the health promoting effects of antioxidants in everyday foods, combined with the negative consumer image of synthetic antioxidants (such as Butylated Hydroxyanisole (BHA), Butylated Hydroxytoluene (BHT), and Tertiary Butyl Hydroquinone (TBHQ) and possible safety issues with synthetic antioxidants, have led to an explosion in the desire to use natural antioxidants in foods. However, many natural antioxidants are expensive, because they are extracted from higher value agricultural commodities such as fruits, spices, and even coffee. A potentially much less expensive source of natural antioxidants would be a process (e.g., waste) stream from current food and beverage processes. An example of such a source is coffee beans.

Because of the food preservation and health benefits associated with AOXs described above, and the increasing awareness of the health benefits of coffee, which includes AOXs, additional uses of coffee are needed. Moreover, a cost effective and natural source of AOXs is desired, especially one that is utilized from a current process.

Document US 2007/286925 A1 relates to a composition for improving eye health.

Document WO 02/071874 A relates to a composition for improving age-related physiological deficits and increasing longevity.

Document WO 2005/074719 A relates to a nutritional composition for improving skin condition and preventing skin diseases.

Document J W Hilton et al., The Canadian veterinary journal, 1989 relates to "antioxidants: function, types and necessity of inclusion in pet foods".

Document K Iwai et al., Journal of agricultural and food chemistry, 2004 relates to "in vitro antioxidative effects and tyrosinase inhibitory activities of seven hydroxycinnamoyl derivatives in green coffee beans".

Document WO 02/085397 A1 relates to a therapeutic preparation from coffee beans and a method for producing the same.

### SUMMARY

The present invention is defined as in claim 1. Further disclosed herein but not part of the present invention is the following:
A pet food composition comprising an antioxidant component derived from a decaffeination stream produced during the decaffeination process of coffee beans. The pet food composition comprises from about 0.002% to about 0.2% of the antioxidant component, by weight of the pet food composition. The pet food composition is able to up-regulate antioxidant enzymes directed off a common transcription element known as the Antioxidant Response Element.

A pet food composition comprising an antioxidant component derived from coffee beans by a method comprising the steps of: providing coffee beans; decaffeinating the coffee beans to produce a decaffeination stream and decaffeinated coffee beans; and processing the decaffeination stream to extract the antioxidant component. The pet food composition comprises from about 0.002% to about 0.2% of the antioxidant component, by weight of the pet food composition.

A pet food ingredient comprises an antioxidant component derived from a decaffeination stream produced during the decaffeination process of coffee beans. The pet food ingredient is selected from the group consisting of meat meals, animal fats, animal digests, animal palatants, fish oils, vitamin pre-mixes, and combinations thereof.

A method of preserving a pet food composition ingredient, the method comprising the steps of: providing a pet food ingredient; proving an antioxidant component derived from a decaffeination stream produced during the decaffeination process of coffee beans; combining the pet food ingredient and the antioxidant component. The pet food composition comprises from about 0.002% to about 0.2% of said antioxidant component, by weight of the pet food composition.

A method of manufacturing a pet food composition, said method comprising the steps of: providing at least one protein ingredient; providing at least one fat ingredient; providing at least one antioxidant component derived from a decaffeination stream produced during the decaffeination process of coffee beans; and combining the protein, the fat and the antioxidant in the manufacture of the pet food composition.

A pet food composition comprising an antioxidant component wherein the antioxidant component comprises Di-CQAs and Mono-CQAs and wherein the Di-CQAs are present at a level greater than the Mono-CQAs.

A preservative system comprising an antioxidant component comprising Di-CQAs and Mono-CQAs wherein the Di-CQAs are present at a level greater than the Mono-CQAs.

### BRIEF DESCRIPTION OF THE DRAWINGS

FIG. 1 depicts a flow diagram of a coffee bean decaffeination process.
FIG. 2 depicts a flow diagram of a coffee bean decaffeination stream post-decaffeination.
FIG. 3 depicts a flow diagram of coffee bean decaffeination and post-decaffeination processes.
FIG. 4 depicts a flow diagram of a coffee bean post-decaffeination process.

### DETAILED DESCRIPTION

For the purpose of promoting an understanding of the principles of the invention, reference will now be made to the embodiments illustrated in the drawings and specific language will be used to describe the same. It will nevertheless be understood that no limitation of the scope of the invention is thereby intended. Such alterations and further modifications in the illustrated device and such further applications of the principles of the invention as illustrated therein as would normally occur to one skilled in the art to which the invention relates are contemplated as within the scope of the invention.

Referenced herein may be trade names for components utilized in some embodiments of the present invention. Embodiments of the invention herein do not intend to be limited by materials under a particular trade name. Equivalent materials (e.g. those obtained from a different source under a different name or reference number) to those referenced by trade name herein may be substituted and utilized in the descriptions herein. Furthermore, referenced herein may be certain brand names of various pieces of equipment used in methods or processing steps. Equivalent pieces of equipments may also be substituted and utilized in the descriptions herein.

It should be understood that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

All percentages and ratios are calculated by weight unless otherwise indicated. All percentages and ratios are calculated based on the total composition unless otherwise indicated.

As used herein, the term "comprising" means various components conjointly employed in the preparation of the compositions of the present disclosure. Accordingly, the terms "consisting essentially of' and "consisting of" are embodied in the term "comprising".

As used herein, the articles including "the", "a" and "an" when used in a claim or in the specification, are understood to mean one or more of what is claimed or described.

As used herein, the terms "include", "includes" and "including" are meant to be non-limiting.

As used herein, the term "companion animal" refers to an animal selected from the group consisting of dog, cat, rabbit, ferret, and gerbil.

As used herein, the term "pet food composition" refers to a composition that can be ingested by a companion animal. Non-limiting examples of pet food compositions include supplements for a companion animal, dog food, cat food, treats, biscuits, raw hide, chews, fillers, gravy, sauce, beverage, supplemental water, and combinations thereof. The pet food composition can be wet, moist and/or dry.

As used herein, the term "complete and nutritionally balanced," unless otherwise specified, refers to a pet food composition having all known required nutrients in proper amounts and proportions based upon the recommendation of recognized authorities in the field of animal nutrition.

As used herein, the term "an antioxidant component," or "antioxidant," means a substance that can delay the onset or slow the rate of oxidation of oxidizable materials. Antioxidant component and antioxidant are used herein interchangeably. An antioxidant is generally a substance that participates in chemical, physiological, biochemical, or cellular processes that inactivate free radicals and/or active-oxygen species (singlet oxygen, hydrogen peroxide, hydroxyl radical, etc.), or prevent free radical-initiated chemical reactions. Antioxidants may exert their effects in two ways: 1) as direct-acting antioxidants that inactivate oxidative agents such as free radicals, and 2) as indirect agents that can modulate the function, activity, or level of other antioxidants or antioxidant mechanisms. Dietary antioxidants are typically reducing agents that can function as a food preservative (BHT, for example) or to protect the body's tissues and fluids from oxidative stress. Antioxidants can be divided into two broad functions. One, as a food preservative, antioxidants are substances that reduce lipid oxidation in foods, extending the shelf-life, palatability, functionality, and nutritional quality of the food. Two, as a biological antioxidant, antioxidants are compounds that protect biological systems against the potentially harmful effects of processes or reactions that cause oxidative stress or damage. Non-limiting examples of antioxidants are polypehnolic compounds, chlorogenic acids, flavonoids, tocopherols, di- or tri-carboxylic acids (such as citric acid), EDTA (ethylene diaminetetracetate), ascorbic acid (vitamin C), anthocyanins, catechins, quercetin, resveratrol, rosmarinic acid, carnosol, Maillard reaction products, enzymes such as superoxide dismutaste, certain proteins, amino acids, and protein hydrolyzates, etc. Several thousand different polyphenols have been identified in foods. The two main types of polyphenols are flavonoids and phenolic acids. Some of the more common flavonoids are quercetin (found in onion, tea, apple), catechin (tea, fruit), hesperidin (citrus fruits), and cyanidin (red fruits). One of the most common phenolic acids is caffeic acid, present in many fruits and vegetables. Caffeic acid, most often esterified with quinic acid as in chlorogenic acid, is the major phenolic compound in coffee. Coffee AOXs, including the chlorogenic acids, and caffeic and ferulic acids, are polyphenolic, hydroxycinnamic acid derivatives found naturally in coffee beans. It is well recognized that green (raw) coffee beans have greater amounts of CGAs than roasted coffee, since roasting decreases the level of intact CGAs. However, other antioxidant compounds, such as the melanoidins, are generated during coffee roasting.

As used herein, the term "decaffeination" means the extraction of caffeine from coffee beans with a solvent. In European Union countries, decaffeinated coffee has a maximum caffeine concentration of 0.1% of the dry mass. In the United States, decaffeinated coffee means less than 3% of the amount initially present in the beans. Decaffeination can include extracting any amount of caffeine from coffee beans, from a very small, negligible amount up to 100% of the caffeine, and all ranges between 0% and 100%.

As used herein, the term "usable form" means that, after decaffeination of coffee beans, the coffee beans remain in a form that can be roasted, ground, and brewed into a coffee beverage that still has the desirable attributes of a consumable, consumer-desired coffee beverage. Decaffeination processes are designed to remove the caffeine but to minimize the removal of any other material that contributes to the typical flavor, color, nutrition, etc. of beverage coffee. For example, most commercial decaffeination is carried out on green coffee beans before roasting so as to minimize flavor and aroma losses. The coffee making process for roast and ground coffee for conventional brewing begins when green coffee beans are roasted to develop the characteristic and expected flavor of coffee. The beans are roasted to a degree (light to dark, for example) that meets the taste expectations of the consumer. Roasted coffee beans are ground and then brewed to produce a liquid drinkable coffee beverage. Although a number of methods to brew coffee exists, a typical procedure is drip brewing, where hot water, which can be at around 180 °F, is added to a basket containing ground coffee (and typically a filter). The hot water extracts flavors, colors, various solids, etc. from the ground coffee, producing the coffee beverage. Well known brewing methods are described in US Patent Nos: 6,808,731; 5,721,005; 6,808,731; and 6,783,791.

As is well known, coffee AOXs, including chlorogenic acids, caffeic, ferulic acids, among others, are present in green coffee, roasted coffee, brewed coffee, and coffee processing streams. At least one of the coffee processing streams includes a stream that is a product of the decaffeination process of green coffee beans. This stream, which is generally a caffeine-laden solvent stream that is a product from the decaffeinated green coffee beans, contains AOXs that can be recovered. Typically, after the caffeine is recovered from this caffeine-laden solvent stream, the remaining stream is discarded.

Chlorogenic acids are polyphenolic, hydroxycinnamic acid derivatives that may be found naturally in coffee beans. CGAs are an ester of caffeic or ferulic acid and quinic acid and are the main phenolic acids in coffee. It is well recognized that green (raw) coffee beans have greater amounts of CGAs than roasted coffee, since roasting decreases the level of intact CGAs. Non-limiting examples of the major chlorogenic acids include: (1) caffeoylquinic acids (CQA) such as 3-CQA, 4-CQA, and 5-CQA; (2) feruloylquinic acids (FQA) such as 5-FQA; and (3) dicaffeoylquinic acids (diCQA) such as 3,4-diCQA, 3,5-diCQA, and 4,5-diCQA.

Chlorogenic acid is an antioxidant both in vivo and in vitro but is not the only antioxidant in coffee. Chlorogenic acids can also be used as food antioxidants, to help preserve flavors, vitamins, lipids, etc. against oxidation. They do so in a manner similar to the classical chemical antioxidants, such as BHT and BHA.

Other coffee compounds have also been shown to have antioxidant properties. For example, the melanoidins, a class of higher molecular weight, brown-colored polymers formed during roasting, have been shown to have radical scavenging activity. Other studies have found that Maillard reaction products, also formed during roasting, also have antioxidant activity.

In addition to characterizing the antioxidants in coffee by their chemical identity (chlorogenic acids, for example), the antioxidants in coffee (or in other foods or beverages) can also be determined by measuring antioxidant activity. Using this approach, it is now well known that coffee delivers a high level of antioxidant activity. For example, the antioxidants in coffee can be measured using an ORAC (oxygen radical absorbance capacity) test, a widely used measure of antioxidant activity in foods and beverages, which a detailed method is described hereinafter. In one form of the ORAC test, a substrate (usually fluorescein) is oxidized by adding a free radical initiator. Coffee, for example, can be assessed for antioxidant activity by measuring the decrease of the oxidation reaction by the addition of coffee. In ORAC and similar tests such as FRAP (ferric reducing antioxidant parameter), TEAC (Trolox equivalent antioxidant capacity), TRAP (total radical trapping antioxidant assay), DPPH (a free radical trapping assay), Total Polyphenols (total reducing capacity measured with Folin-Ciocalteu assay), and LDL oxidation (low density protein oxidation assay), coffee has been shown to have a high degree of antioxidant activity.

Thus, it is desirable to recover antioxidants from the coffee decaffeination process. It is also desirable that the amount of antioxidants recovered is usable in other products, which are described hereinafter in just some embodiments. It is further desirable that the coffee decaffeination process is designed to remove the caffeine of the coffee bean but to minimize the removal of any other material that contributes to the typical flavor, color, nutrition, etc. of any coffee products derived therefrom.

An antioxidant component is produced or recovered during the decaffeination process of coffee beans. Caffeine is a physiologically active component in coffee, and coffee beans contain between 0.8 and 2.8% caffeine, depending on their species and origin. Coffee bean decaffeination is conducted, at least in part, to remove part of the caffeine from coffee beans. It can be desirable to produce coffee beans with a lower amount of caffeine that are then roast and ground because roast and ground coffee with lower amounts of caffeine is a desirable product that is bought by the consuming public.

Only negligible losses of caffeine occur during the roasting process. In order to minimize the 'negative' physiological effects from the caffeine, and still maintain the desirable attributes of a coffee beverage, many decaffeination processes have been developed. To minimize flavor and aroma losses, commercial decaffeination of coffee can be performed on the green coffee beans before roasting.

However, before describing the details related to green coffee beans, it should be understood that the process to recover an antioxidant described herein (but not forming part of the present invention) is not limited to recovery from coffee beans. Other sources of antioxidants are within the scope of the present application. Sources include naturally caffeinated products, or natural caffeine containing products. Such products can include coffee beans, tea leaves, cocoa beans, chocolate products, guarana, and mixtures and combinations of these. These natural caffeine containing products can be decaffeinated by decaffeination processes that are well known in the art. After decaffeination, the then decaffeinated natural caffeine containing product can remain in a usable form for making a consumer product. Consumer products can include brews of roasted and ground coffee, tea, black tea, green tea, chocolate, and mixtures and combinations thereof. Thus, it should be understood that while the description and examples hereinafter discuss coffee beans, these additional natural caffeine containing products are also envisioned.

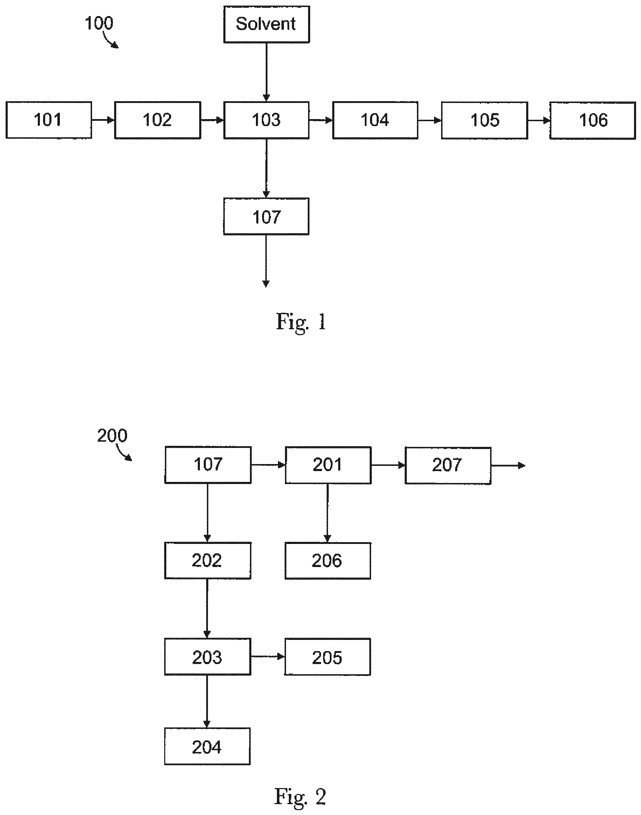

Coffee bean decaffeination generally involves several following steps, which have been at least partially described in the followed US Patents: 3,671,262; 3,671,263; 3,700,464; 4,256,774; 4,279,937; 4,474,821, all assigned to The Procter & Gamble Company of Cincinnati, Ohio. FIG 1 illustrates just one set of steps of a typical decaffeination process, shown as flow chart 100. These steps include beginning with providing coffee beans, such as raw, green coffee beans. Raw, green coffee beans 101 can then be wetted with water to swell the beans in step 102. Wetting can occur with steam. This wetting also makes the caffeine more available for extraction. After wetting, caffeine can then be extracted from the coffee beans with a solvent, shown in step 103, of which one non-limiting example includes ethyl acetate. Non-limiting examples of other solvents can include water and supercritical carbon dioxide. Some solvents can include solvents that are classified as "natural" solvents. As used herein, one definition of "natural" can be the definition used by pet food regulators, such as the American Association of Feed Control Officials (AAFCO). AAFCO's definition of "natural" is: "A feed or ingredient derived solely from plant, animal, or mined sources, either in its unprocessed state or having been subject to physical processing, heat processing, rendering, purification, extraction, hydrolysis, enzymolysis, or fermentation, but not having been produced by or subject to chemically synthetic process and not containing any additives or processing aids that are chemically synthetic except in amounts as might occur unavoidably in good manufacturing practices." As mentioned, the coffee beans can first be decaffeinated with a solvent, such as ethyl acetate, and this caffeine-laden solvent can be referred to as the "spent solvent." Without being bound by theory, it is believed that use of ethyl acetate as a decaffeinating solvent will result in a decaffeination stream with a pH from about 2 to about 4 due to the breakdown products of ethyl acetate which are acetic acid and ethanol. Typically the "solvent" can then be recovered and reused in the decaffeination process, and the resulting mostly aqueous stream can then be further processed to recover caffeine.

The coffee beans can then be steam-stripped in step 104 to remove any residues that have been left from the solvent. The coffee beans can then be dried in step 105. After drying, the coffee beans are then generally in a suitable form for roasting, grinding, and brewing into a conventional coffee beverage, as represented by step 106.

The decaffeination solvent stream, shown in step 107 of FIG. 1, contains an antioxidant component comprising the polyphenol chlorogenic acid and can contain caffeine and a small amount of other dissolved coffee solids, non-limiting examples of which include carbohydrates, and flavor precursors, which are inadvertently removed during the decaffeination process. A variety of processes exist and are well known in the art to handle this decaffeination solvent stream 107, one non-limiting example of which is illustrated by FIG. 2. The solvent itself can be recovered/recycled and used for further decaffeination processing, as shown in FIG 2. The solvent and extracts stream 107 can be split into a waste stream 201 and a solvent/solids stream 202. Often the decaffeination process also includes the recycling of any solids (other than caffeine) back into the decaffeination process, which can be represented by steps 203, 204, and 205 of FIG. 2. This recycling can include the antioxidants such as polyphenols like chlorogenic acids, for example. The solvent of 204 can be separated from the solids of 205. The solvent 204 can then be recycled back into the decaffeination process to be used for further extraction of caffeine from coffee beans. The remaining solids in 205 can then be recycled back into the already decaffeinated coffee. Typically, the caffeine and other coffee solids and waste products are removed from the solvent stream, illustrated by 201. The caffeine of this stream may be recovered and sold for food, pharmaceutical use, or otherwise, as shown in 206. The material, liquid and/or solid, remaining after the removal of the caffeine, solvent, and the recovery of any other solids, still containing some caffeine and other coffee solids, is usually discarded, shown by 207, and is generally termed "waste stream," "decaf waste stream," or "waste material" by those of ordinary skill in the art. However, this waste material, in addition to containing caffeine not removed by the recovery process, also contains coffee solids and antioxidants comprising polyphenols including chlorogenic acids that were inadvertently removed during decaffeination and were not recycled back into the decaffeination process. Moreover, as mentioned above, each of the streams derived from stream 107 contains at least some amount of coffee solids and antioxidants which comprise chlorogenic acid.

Stream 107 can be processed using various steps to recover caffeine, solvent, and/or other materials from the decaffeination solvent. Non-limiting examples of processes used for recovery include evaporation, separation, liquid-liquid extraction, water stripping, crystallization, centrifugation, etc., and other processes known to those skilled in the art.

It should be understood that the primary purpose of coffee decaffeination is to remove caffeine from coffee beans but otherwise leave the beans as unchanged as possible. In other words, decaffeination is designed to remove as much caffeine as possible but as little as possible of the other coffee solids (See: W. Heilmann. 2001. "Technology II: Decaffeination of Coffee." Chapter 5 in Coffee: Recent Developments, edited by R.J. Clarke and O.G. Vitzthum, Blackwell Science, London). These other coffee solids include carbohydrates, flavor precursors, amino acids, and antioxidants such as polyphenols like chlorogenic acids. For example, decaffeination is designed to remove as few chlorogenic acids as possible because chlorogenic acids are critical to the flavor of coffee. In addition, economic concerns play a role when solids are removed from the coffee bean as volume and weight has been decreased. As a result of minimizing the removal of coffee solids other than caffeine itself, the decaffeinated beans can be roasted and used to make a good-tasting, consumer acceptable cup of (decaffeinated) coffee. Minimizing the loss of coffee solids (other than caffeine) in decaffeinated coffee is typically done by minimizing the extraction of such solids during decaffeination itself, and/or by "adding back" such solids to the decaffeination process and beans, which has been shown in FIG 2.

However, during decaffeination, as recognized and pointed out above, other coffee solids compounds are also removed inadvertently from the coffee bean, including a small portion of antioxidants comprising chlorogenic acids. The decaffeination solvent and dissolved coffee solids are typically recovered and recycled back into the decaffeination process, the caffeine is typically recovered and used in other applications. In many decaffeination processes, the coffee solids inadvertently removed during decaffeination are also recycled back into the decaffeination process to minimize the loss of such solids from the decaffeinated beans. For example, the most selective process for removing just caffeine and not other coffee solids is by using supercritical carbon dioxide. Decaffeination with carbon dioxide provides a product of high quality because the loss of coffee solids (other than caffeine) is quite low. Other decaffeination processes that minimize loss of non-caffeine coffee solids during decaffeination are also well known. Thus, decaffeination as used today is meant to maximize the removal of caffeine while leaving coffee solids in the decaffeinated coffee beans.

The current processes known in the art that remove antioxidants from coffee beans render the resulting coffee bean unusable for brewing. However, after the decaffeination processes described above, which result in a recoverable amount of antioxidants such as polyphenols like chlorogenic acid, and according to embodiments of the present invention, the decaffeinated green coffee beans can be in a usable form and can be used thereafter. Non-limiting examples of some uses include roasting and grinding for conventional brewing, converting into instant coffee in the same manner as non-decaffeinated coffee beans, and preparation of an extract for use in ready-to-drink beverages and other extract uses. The composition of the decaffeinated coffee, apart from caffeine content, is very similar to caffeinated (regular) coffee. Of course, slight differences do exist in composition and flavor depending on the particular decaffeination process employed. A comparison of the proximate composition of caffeinated (regular) coffee to decaffeinated coffee shows that other than caffeine content, decaffeinated coffee is quite similar to regular coffee in composition. The amount of caffeine in green coffee is approximately 1-2% (dry wt. basis), depending upon the species (See: K. Ramalakshmi and B. Raghavan, 1999, Caffeine in coffee: Its removal. Why and How? CRC Critical Reviews in Food Science and Nutrition, 39 (5): 441-56). In European Union (EU) countries, by definition, a decaffeinated coffee means a maximum caffeine concentration of 0.1% (dry weight basis). In the United States (US), by definition, a decaffeinated coffee means less than 3% of the caffeine amount initially present in the beans is present in the resulting decaffeinated coffee. Therefore, in the EU, a minimum of 90-95% of the caffeine has been removed from coffee during decaffeination (assuming 1-2% caffeine in green coffee). In the US, a minimum of 97% of the caffeine has been removed during decaffeination.

**Table A. Proximate Composition of Decaffeinated and Regular Coffee***

| Material (%, proximate composition) | Regular (Caffeinated), untreated Coffee | Decaffeinated Coffee (methylene chloride extracted) | Decaffeinated Coffee (supercritical carbon dioxide extracted) |
|---|---|---|---|
| Crude Protein | 14.2 | 14.5 | 14.8 |
| Tannins | 10.3 | 10.1 | 11.0 |
| Petroleum ether extract | 9.7 | 4.5 | 6.0 |
| Total Ash | 4.5 | 4.3 | 4.4 |
| Total Nitrogen | 2.6 | 2.2 | 2.3 |
| Caffeine | 2.1 | 0.05 | 0.06 |

| | | | |
|---|---|---|---|
| Source: Ramalakshmi and Raghavan, 1999, Caffeine in Coffee: Its Removal, Why and How? Critical Reviews in Food Science and Nutrition, 39: 441-56. | | | |

In addition to similar proximate compositions, decaffeinated and regular (caffeinated) coffee are similar in carbohydrates, acids, lipids, proteins, amino acids, chlorogenic acids, colors, and volatiles like flavor compounds. Table B shows that decaffeinated coffee beans contain essentially the same levels and kinds of chlorogenic acids as caffeinated beans. Again, this result of levels is not surprising since the decaffeination process is designed to minimize the removal of coffee solids other than caffeine.

**Table B. Chlorogenic Acids in Green Coffee Before and After Decaffeination**

| | - - - - - - - - - g/kg dry matter - - - - - - - - - - - - | | | |
|---|---|---|---|---|
| Coffee Samples | mono-CQAs | mono-FQAs | di-CQAs | Total CGAs |
| Coffee A, before decaf | 60.4 | 9.8 | 13.6 | 83.8 |
| Coffee A, after decaf | 59.1 | 7.6 | 11.4 | 78.1 (-6.8%) |
| Coffee B, before decaf | 68.7 | 11.2 | 15.5 | 95.4 |
| Coffee B, after decaf | 68.7 | 8.8 | 13.3 | 90.8 (-4.8%) |

| | - - - - - - - - - - - - % of total CGAs - - - - - - - - - | | | |
|---|---|---|---|---|
| Coffee A, before decaf | 72.1% | 11.7% | 16.2% | |
| Coffee A, after decaf | 75.7% | 9.7% | 14.6% | |
| Coffee B, before decaf | 72.0% | 11.7% | 16.2% | |
| Coffee B, after decaf | 75.7% | 9.7% | 14.6% | |

| | | | | |
|---|---|---|---|---|
| Coffee samples A and B are two separate samples from a commercial decaffeination operation. | | | | |

Table B shows that the difference between the total chlorogenic acids in a decaffeinated Coffee A and a caffeinated Coffee A is about 5.7g/kg, which equates to about a 6.8% decrease in total chlorogenic acids after decaffeination. Table B also shows the difference in total chlorogenic acids between decaffeinated Coffee B and caffeinated Coffee B is about 4.6 g/kg, which equates to about a 4.8% decrease in total chlorogenic acids after decaffeination. In three scientific publications, a loss of 4.8-11% of the total chlorogenic acids occurred during decaffeination when measuring the coffee bean (See: A. Farah & CM Donangelo, 2006, Phenolic compounds in coffee, Brazilian J. Plant Physiol., 18(1): 23-26; MN Clifford, chapter 5 "Chlorogenic Acids" in RJ Clarke & R Macrae, editors, 1985, Coffee, Volume 1, Chemistry, Elsevier Applied Science, NY; and Moreira et al., 2005, Contribution of chlorogenic acids to the iron-reducing activity of coffee beverages, J. Agric. Fd. Chem. 53: 1399-1402).

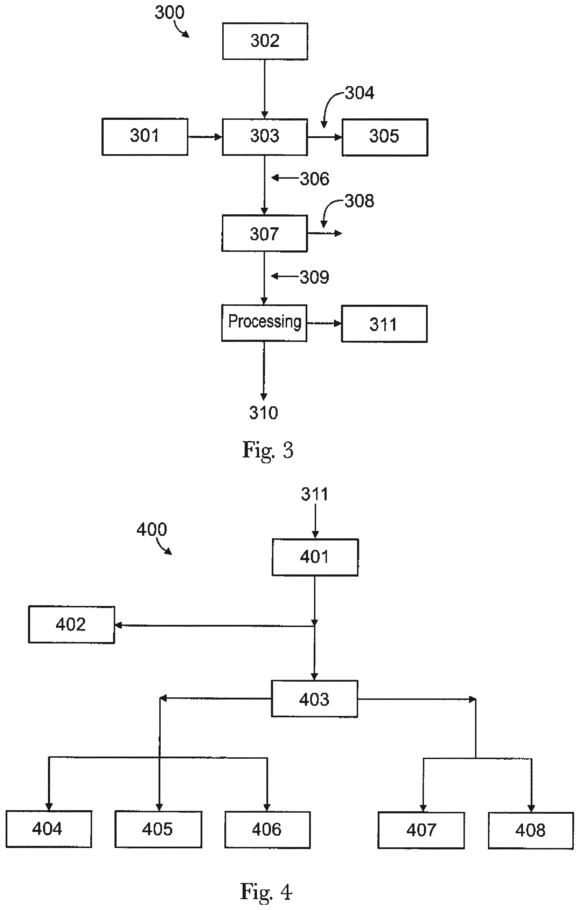

Looking back to FIG. 2, two decaffeination streams can contain recoverable antioxidants: the decaffeination solvent stream 107 directly resulting from the decaffeination of the green coffee beans, and the waste stream 207 remaining after recovery of solvent and caffeine. As described above, the decaffeination solvent stream 107 can contain recoverable materials including the caffeine and the solvent. Waste stream 207 can also include other recovery materials, such as coffee solids, non-limiting examples of which include antioxidants such as polyphenols like chlorogenic acid. Thus, a process flow diagram 300 of FIG. 3 can be used to illustrate the combined aspects of FIGs 1 and 2. Again, caffeine from coffee beans 301 is extracted with solvent 302 in decaffeination process 303. The result can include usable beans 304, which can then be further processed in 305 and sent for making consumer acceptable roast and ground coffee for brewing, instant coffee, or a coffee extract. The decaffeination solvent stream 306, akin to 107 from process 100, can be removed from the coffee beans and can be sent to a solvent separator process 307. Separation can be performed such that a solvent stream 308, which can be a first solvent stream, and a caffeine stream 309, which can be a second caffeine stream, result, akin to 201 and 202 from process 200. This caffeine stream 309 can be further processed, as hereinafter described, to remove the caffeine in stream 310, or a third stream, and leave a waste stream 311, or fourth stream, which can be a source of antioxidants, such as polyphenols like chlorogenic acid.

In one sample that was analyzed from the decaffeination solvent stream 306, and as shown in Table C, the sample was shown to contain predominantly caffeine, along with a much smaller proportion of other coffee solids that included chlorogenic acids. The ratio of caffeine to the non-caffeine solids in this particular sample of the decaffeination solvent stream was about 3:1, which means that the ratio of caffeine to chlorogenic acids would be at least 3:1, since other coffee solids other than chlorogenic acids could also be included.

**Table C. Composition of Decaffeination Solvent Stream**

| Composition | Decaffeination Solvent Stream |
|---|---|
| Total Solids (%) | 0.771 |
| Caffeine (%) | 0.576 |
| Non-Caffeine Solids (%)* | 0.195 |

| | |
|---|---|
| Decaffeination solvent stream sample recovered from commercial decaffeination process using ethyl acetate as solvent. *Difference between total solids and caffeine. | |

The level or amount of chlorogenic acids in the decaffeination solvent stream is shown in Table D for samples A and B. The results shown in Table D, expressed in gram per Liter, show that less than one (1) gram chlorogenic acids per Liter solvent was measured. These results would not lead one to conclude that a high level of chlorogenic acids is available for recovery and use. Two separate samples, A and B, were taken from the decaffeination solvent stream of one example.

**Table D. Chlorogenic Acids in the Decaffeination Solvent Stream**

| Chlorogenic Acids | Spent Solvent A | Spent Solvent B |
|---|---|---|
| | - - - - - - g/L - - - - - - - - | |
| Mono-CQAs | 0.0924 | 0.145 |
| Mono-FQAs | 0.078 | 0.207 |
| Di-CQAs | 0.129 | 0.451 |
| Total CGA's | 0.299 | 0.803 |

| | - - - - - - - % of total CGA's - - - - - - - - | |
|---|---|---|
| Mono-CQAs | 30.9% | 18.1% |
| Mono-FQAs | 26.1% | 25.8% |
| Di-CQAs | 43.1% | 56.2% |

| | | |
|---|---|---|
| Solvent samples A and B recovered from a commercial decaffeination process using ethyl acetate as solvent. | | |

From comparing Table B to Table D, it is shown that of the chlorogenic acids, the amount of Di-CQAs (of the total CGAs) has increased, or been enriched, as shown by an increase from 16.2% to 43.1% and 16.2% to 56.2%, in Coffee A/Solvent A and Coffee B/Solvent B, respectively. Thus, one of ordinary skill in the art can conclude that Di-CQAs, when compared to the total CGAs, have been enriched or concentrated from the coffee bean to the decaffeinated solvent stream. In an embodiment, the Di-CQAs are present in an amount greater than the Mono-CQAs. In an embodiment, the ratio of Di-CQAs to Mono-CQAs is from about 3:1 to about 1.3:1.

In one sample of the waste stream that was analyzed and evaluated, the composition was determined to include a high proportion of caffeine and chlorogenic acids, as shown in Table E. The waste stream also contained a significant level of antioxidant activity as measured by the ORAC test. The level of total polyphenolic compounds in the waste stream, about 20%, closely matched the level of chlorogenic acids in the waste stream, about 24%, which was expected because the chlorogenic acids account for essentially all the polyphenols in green coffee.

**Table E. Composition of Waste Stream***

| Measurement | Waste Stream* |
|---|---|
| Total Solids | 11.0% |
| Caffeine | 47.1 % of solids |
| Chlorogenic Acids | 23.8% of solids |
| Total Polyphenols | 201 mg polyphenols/gram dry wt. |
| ORAC Antioxidant Activity | 4.4 mmoles Trolox equiv/gram dry wt. |

| | |
|---|---|
| *Waste stream sample A recovered from commercial ethyl acetate decaffeination process after recovery of solvent and caffeine from the decaffeination solvent stream. | |

Two samples of the waste stream were analyzed for chlorogenic acids and found to contain 14-26 grams chlorogenic acids/Liter waste stream, as shown in Table F.

**Table F. Chlorogenic Acids in the Waste Stream**

| Composition | Waste Stream A | Waste Stream B |
|---|---|---|
| Solids | 11.02% | --- |
| Mono-CQA's (g/L) | 11.6 | 3.94 |
| Mono-FQA's (g/L) | 8.38 | 4.03 |
| Di-CQA's (g/L) | 5.57 | 6.26 |
| Total CGA's (g/L) | 25.6 | 14.2 |
| Caffeine (g/L) | 51.8 | 50.0 |
| Mono-CQA's (% of total CGA's) | 45.3% | 27.7% |
| Mono-FQA's (% of total CGA's) | 32.7% | 28.4% |
| Di-CQA's (% of total CGA's) | 21.8% | 44.1% |

| | | |
|---|---|---|
| Waste Stream samples A and B were recovered from commercial ethyl acetate decaffeination process, after recovery of solvent and caffeine from spent solvent stream. | | |

In relation to the level of caffeine, the total amount of chlorogenic acids in the waste stream is lower that than in a coffee beverage (Table G). For example, the waste stream had about 7-times the concentration of caffeine as regular coffee, but only about 3-times the concentration of total CGA's (Table G). Comparing the relative amounts of the individual chlorogenic acids in the waste stream to that in a coffee beverage (decaffeinated or regular), the waste stream is especially enriched in the FQAs and the di-CQAs, as shown in Table G. This may account in part for the antioxidant potency of the waste stream because the di-CQA's have been reported to have higher antioxidant activity than the CQA's (K. Iwai et al., 2004, In vitro Antioxidative Effects and Tyrosinase Inhibitory Activities of Seven Hydroxycinnamoyl Derivatives in Green Coffee Beans, J. Agric. Food Chem. 52: 4893-8). The lower amount of CQA in the waste stream may reflect greater complexation of the CQA with caffeine and subsequent removal when the caffeine is recovered.

**Table G. Chlorogenic Acids in Waste Stream Relative to Coffee**

| | - - - - - - - - - - - - - - - - - - - - - - **ppm** - - - - - - - - - - - - - - - - - - - - - - | | | | | Ratio of CGA/Caffeine |
|---|---|---|---|---|---|---|
| Material | CQA | FQA | di-COA | Total CGA | Caffeine | |
| Waste Stream A | 1048 | 760 | 506 | 2314 | 4703 | 0.49 |
| Regular Coffee | 622 | 98 | 69 | 789 | 692 | 1.14 |
| Decaf Coffee | 668 | 103 | 69 | 839 | 65 | 12.9 |

| | - - - - - - - - - - - - - - - - - **% of Total CGA** - - - - - - - - - - - - - - - - - | | | | | |
|---|---|---|---|---|---|---|
| Material | CQA | FQA | di-COA | Total CGA | | |
| Waste Stream A | 45.3 | 22.9 | 21.8 | 100 | | |
| Regular Coffee | 78.8 | 12.4 | 8.7 | 100 | | |
| Decaf Coffee | 79.6 | 12.3 | 8.2 | 100 | | |

| | | | | | | |
|---|---|---|---|---|---|---|
| Waste Stream A recovered from commercial ethyl acetate decaffeination process. Regular coffee = commercial sample of Folgers Classic. Decaf coffee = commercial sample of Decaf Folgers Classic. | | | | | | |

Another way to evaluate the antioxidants, or antioxidant activity, in a material such as the waste stream, in addition to quantifying the levels of the polyphenolic antioxidants (the amount of CQA, for example), and in addition to measuring antioxidant activity in a chemically-based assay (such as the ORAC test), is to measure a biological/physiological effect of the antioxidant material. In one example, usage of the waste stream up-regulated the genes responsible for antioxidant enzymes in the cell. Cells are exquisitely sensitive to an imbalance in anti-oxidative capacity and respond to this imbalance by up-regulation of a family of antioxidant proteins that are coordinately regulated by transcription off the Antioxidant Response Element (ARE). Some of these proteins increase the antioxidant capacity of the cell and others detect damage and facilitate repair of oxidative damage. This mechanism of increasing the anti-oxidative potential of cells is, therefore, distinctly different than the ability of the waste stream to directly reduce chemical oxidants such as is measured by the chemically-based ORAC assay. An Antioxidant Response Element (ARE) reporter cell line (human breast epithelial cells) from CXR Biosciences was used to assay for the increased transcription off the ARE. An upregulation of antioxidant enzymes, as demonstrated by the increased transcription of the ARE, can provide cellular immune defense, increase the antioxidant capacity of the cell, detect damage, and facilitate repair of oxidative damage. Cells were treated overnight with a dilute solution of the waste stream and then assayed for the increase in activation off the ARE (see specific method below). The waste stream strongly induced the ARE (endogenous antioxidant enzymes), similar to brewed coffee, as shown in Table H. When a small amount of the waste stream was added to coffee grounds, an increase in the ARE activity of the coffee beverage brewed from those grounds occurred. These data illustrate that the waste stream is able to regulate transcription of the genes responsible for increasing the antioxidant capacity of the cell - essentially turning on the cells own antioxidant defense system.

**Table H. Induction of Endogenous Cellular Defenses (ARE) by Waste Stream**

| | |
|---|---|
| Material (% solids) | ARE Response (% increase relative to control) |
| Waste Stream (1.0%) | 587 |
| Brewed Coffee (0.76%) | 686 |
| Brewed Coffee + 20 ml Waste Stream (0.92%) | 737 |

| | |
|---|---|
| Cells in ARE (antioxidant response element) test were treated with a 1% solution of the waste stream, or the coffee, at a 5% treatment level of the cells. Waste stream sample A. "Brewed Coffee" was a sample of commercial, caffeinated, roast & ground coffee that was brewed as described below in Table J. | |

As mentioned above, the waste stream 311 can be further processed to recover and increase the concentration and purity of the antioxidants. This further processing to recover antioxidants can be chosen from or include several methods known in the art. Non-limiting examples include chilling, evaporation, rotary evaporation, wiped film evaporation, drying, vacuum drying, spray drying, freeze drying, extraction with a solvent, extraction with ether, acidification, distillation, centrifugation, concentration, filtration, ultra filtration, chromatographic separation, column separation, and mixtures and combinations. An example of such processing is outlined in FIG. 4 and represented by process flow diagram 400. First, the waste stream 311 can be processed at 401 by chilling, and solids can be formed and precipitated and recovered by filtration in step 402. Then, the filtrate 403 that is separated from the solids, which can contain most of the total solids from the waste stream, can be even further processed. In one example, the solids were slightly enriched in chlorogenic acids, polyphenols, and ORAC antioxidant activity compared to the waste stream, as shown in Table I. In this single example, the ratio of chlorogenic acid to caffeine increased to 0.66 in the solids compared to 0.51 in the waste stream.

**Table I. Antioxidant Content in Processed Waste Stream**

| Process Step | Total CGAs | Total PPs | ORAC | Caffeine | Ratio CGA/Caffeine |
|---|---|---|---|---|---|
| 1. Waste Stream | 23.8 | 201 | 4.4 | 47.1 | 0.51 |
| 2. Solids | 28.2 | 293 | 5.8 | 42.6 | 0.66 |
| 3. Filtrate | 16.4 | 172 | 4.6 | 49.7 | 0.33 |
| 4. Vacuum Dried | 4.9 | 44 | 3.6 | 32.8 | 0.15 |
| 5. Rotovap Concentrated | 17.1 | 178 | 4.6 | 49.6 | 0.34 |
| 6. Freeze Dried | -- | -- | 4.6 | -- | -- |
| 7. Ether Extract | 3.4 | 159 | 7.2 | 27.9 | 0.12 |
| 8. Acidified Ether Extract | 5.7 | 264 | 9.3 | 5.8 | 0.98 |

| | | | | | |
|---|---|---|---|---|---|
| CGAs = chlorogenic acids (%, dry wt. basis). PPs = polyphenols (mg/gram). ORAC = oxygen radical absorbance capacity (mmoles Trolox equivalent/gram). Caffeine (% dry wt. basis). | | | | | |

Even further processing can include the filtrate 403 being concentrated by rotary evaporation 404, or dried in a vacuum oven 405 or by freeze drying 406. Conditions for these processes are as known to those of ordinary skill in the art. In one example, the sample dried in the vacuum oven had lower CGAs, PPs, and ORAC activity, presumably due to degradation of the antioxidants during this treatment step. The rotary evaporation sample had a composition essentially equal to the filtrate, indicating no degradation during this step. The freeze dried sample had ORAC antioxidant activity the same as the filtrate, indicating no antioxidant degradation during freeze drying. It should be understood that any one of these processing steps can be done either alone or in combination with the others. As another example, concentration may occur by use of plate evaporators. For example, a five-effect plate evaporator (i.e., such as available from GEA Niro Inc.) is directly heated causing evaporation and condensing the filtrate 403. As another example, concentration may occur by use of a vacuum evaporator.

Alternatively, the filtrate 403 can be extracted with ether, either before or after acidification of the filtrate, represented by streams 407 and 408, respectively. In one example, the acidified ether extract of the filtrate had the highest ratio of chlorogenic acid to caffeine in all samples, 0.98, and the highest ORAC activity, indicating a promising route to purification.

The antioxidant material recovered from the decaffeination process can be used for many purposes, non-limiting examples of which include as a food preservative or to increase the antioxidant activity in a food or beverage. Those skilled in the art will recognize that the antioxidants derived from coffee decaffeination are widely applicable to foods and beverages.

In one coffee example, the waste stream, added to coffee before or after brewing, increased the antioxidants and AOX activity in the coffee, as shown in Table J. A sample of regular caffeinated commercial roast and ground coffee was brewed (40 g coffee, 1420 ml water, in a conventional drip brewer) and evaluated for antioxidants and AOX activity. When 20 ml of the waste stream was added to the coffee, either to the ground coffee before brewing or to the beverage coffee after brewing, an increase in ORAC antioxidant activity of about 28% (from 7.1 to 9.1 or 9.2, as in Table J) occurred. Increases in total chlorogenic acids and total polyphenols also existed, as shown in Table J.

Also shown in a footnote to Table J is an ORAC range of about 3 to about 8 for regular coffee, brewed in the same manner (40 g coffee and 1420 ml water in a conventional drip brewer). Thus, regular coffee can have an ORAC value in the range of about 3-8. Thus, as shown from Table J, when adding an amount of waste stream to regular coffee, ORAC can increase by at least about 28%. Of course, it should be understood to those skilled in the art that the AOX level and thus AOX activity can be impacted by adding more or less of the waste stream to a coffee product or any other product.

**Table J. The Waste Stream Increases the Antioxidants in Brewed Coffee**

| Material | ORAC | Total CGAs | Total PPs | ARE |
|---|---|---|---|---|
| Regular Coffee | 7.1 | 732 | 1383 | 686 |
| Regular Coffee + 20 ml WS* | 9.1 | 779 | 1604 | 737 |
| Regular Coffee + 20 ml WS** | 9.2 | 885 | 1651 | --- |

| | | | | |
|---|---|---|---|---|
| ORAC = oxygen radical absorbance capacity (mmoles/8 ounces). Total CGAs (chlorogenic acids) and Total PPs (polyphenols) are in ppm. "Regular Coffee" was a sample of commercial, caffeinated, roast and ground coffee that was brewed as follows: 40 g coffee and 1420 ml water in a conventional drip brewer. * 20 ml of the waste stream (sample A) was added to the ground coffee before brewing. ** 20 ml of the waste stream (sample A) was added to the brewed coffee beverage after brewing. ARE = % increase in antioxidant response element relative to control. Solids content (g/100 ml) of regular coffee (0.76), coffee + WS* (0.92), and coffee + WS** (0.94). Note: we found a range in ORAC of 3.2-7.9 mmoles Trolox/8 ounces (average 5.6) for 30 different samples of commercial, caffeinated, roast and ground coffees that were brewed as described above (40 g coffee and 1420 ml water in a conventional drip brewer). The 30 samples of coffee represented a range of brands, roast degree, and bean type. | | | | |

The pet food composition can be selected for consumption by an animal, such as a companion animal, while not intended for consumption by a human. Non-limiting examples of pet food compositions include supplements for an animal, dog food, cat food, treats, biscuits, raw hide, chews, fillers, gravy, sauce, beverage, supplemental water, and combinations thereof. The pet food composition herein can be complete and nutritionally balanced. A complete and nutritionally balanced pet food composition may be compounded to be fed as the sole ration and is capable of maintaining the life and/or promote reproduction without any additional substance being consumed except for water.

The antioxidant component can be a preservative system. The antioxidant component can be used to preserve at least one ingredient in a moist pet food composition (i.e., those pet food compositions having a total moisture content of from about 16% to about 50%, by weight of the product), and/or wet pet food composition (i.e., those having a total moisture content of greater than about 50%, by weight of the product), and/or dry pet food composition (i.e., those having a total moisture content of from about 0% to about 16%, by weight of the product). Unless otherwise described herein, wet pet food composition, moist pet food composition and/or dry pet food composition are not limited by their composition or method of preparation.

The antioxidant component as used in the present invention is derived from a decaffeination waste stream produced during the decaffeination process of coffee beans and comprises chlorogenic acid.

The antioxidant component derived from a decaffeination stream produced during the decaffeination process of coffee beans and which comprises chlorogenic acid is utilized to preserve at least one ingredient within a pet food composition. Generally, the antioxidant component is utilized to preserve at least one ingredient in a pet food composition that may be categorized as at least one of the following: meat meals, animal fats, animal digests, animal palatants, fish oils, vitamin pre-mixes, and combinations thereof. Non-limiting examples of pet food composition ingredients that can be preserved by an antioxidant component recovered from a decaffeinated stream produced during the decaffeination process of coffee beans include chicken, chicken meals, chicken by-product means, chicken digests, lamb, lamb meals, turkey, turkey meals, beef, beef by-products, beef meals, pork, pork by-products, pork meals, animal by-products, venison, venison by-products, fish digests, viscera, entrails, chicken fat, turkey fat, beef fat, poultry lard, tallow, fish oil, menhaden oil, anchovy oil, fresh meat, vitamin pre-mixes, fat-based palatants, and combinations thereof.

A pet food composition (not per se part of the present invention) comprising an antioxidant component as described herein may comprise from about 0.01, 0.02, 0.03, 0.04, 0.05, 0.06, 0.07, 0.08, 0.09, 0.1, 0.15, 0.18, 0.2 or 0.25 grams of antioxidant component per kilogram of pet food composition to about 0.3, 0.35, 0.37, 0.4, 0.5, 0.7, 0.9, 1.0, 1.2, 1.4, 1.6, 1.8, 2.0 or 2.5 grams of antioxidant component per kilogram of pet food composition. Meat meals of a pet food composition may comprise from about 30, 60, 150, or 300 ppm to about 500, 600, 1500, or 3000 ppm of antioxidant component per kilogram of meat meal. A pet food composition as described herein may comprise about 300, 750, or 1500 ppm antioxidant component per kilogram of meat meal. Animal fats of a pet food composition may comprise from about 90, 180, 450 or 900 ppm to about 1500, 1800, 4500, or 9000 ppm of antioxidant component per kilogram of animal fat. A pet food composition as described herein may comprise about 900, 1800, or 4500 ppm of antioxidant component per kilogram of animal fat. Animal digests and animal palatants of a pet food composition may comprise from about 5, 10, 25 or 50 ppm to about 75, 100, 500 or 1000 ppm of antioxidant component per kilogram of animal digests and animal palatants. A pet food composition as described herein may comprise about 50, 250 or 500 ppm antioxidant component per kilogram of animal digest and animal palatants. Fish oils of a pet food composition may comprise from about 120, 240, 600, or 1200 ppm to about 2000, 2400, 6000, or 12000 ppm of antioxidant component per kilogram of fish oils. A pet food composition as described herein may comprise about 1200, 3000 or 6000 ppm of antioxidant component per kilogram of fish oils. Vitamin pre-mixes of a pet food composition may comprise from about 2400, 4800, 12000, or 24000 ppm to about 48000, 120000, or 240000 ppm of antioxidant component per kilogram of vitamin pre-mix. A pet food composition as described herein may comprise about 24000, 60000, 120000 ppm of antioxidant component per kilogram of vitamin pre-mix.

A pet food composition as described herein may comprise from about 0.002, 0.004, 0.008 or 0.01% to about 0.04, 0.08, 0.1, 0.12, 0.14, 0.16, 0.18 or 0.2% antioxidant component by weight of the pet food composition. In a disclosure in which the pet food composition is a biscuit, the pet food composition may comprise from about 0.002, 0.004, or 0.01 to about 0.04, 0.1, or 0.2% antioxidant component by weight of the pet food composition.

In addition to proteinaceous and farinaceous materials, the pet food composition generally may include vitamins, minerals, and other additives such as flavorings, preservatives, emulsifiers and humectants. The nutritional balance, including the relative proportions of vitamins, minerals, protein, fat and carbohydrate, is determined according to dietary standards known in the veterinary and nutritional areas.

Non-limiting examples of dry pet food compositions comprise, on a dry matter basis, from about 1, 5 or 10% to about 25, 35 or 50% crude protein, from about 0.5, 5 or 10% to about 20 or 25% crude fat, from about 1, 2 or 3% to about 6, 8 or 10% supplemental fiber, from about 1, 2 or 5% to about 15, 20 or 30% moisture, all by weight of the pet food composition. In an embodiment, the dry pet food composition comprises, on a dry matter basis, a minimum protein level of about 9% to about 22%, a minimum fat level of about 8% to about 13%, a minimum moisture level of about 3% to about 8%, a minimum supplemental fiber level of about 3% to about 7%, all by weight of the pet food composition.

Non-limiting examples of wet pet food compositions comprise, on a dry matter basis, from about 0.5, 5 or 10% to about 25, 35 or 50% crude protein, from about 0.5, 5 or 10% to about 20 or 25% crude fat, from about 0.01, 0.05, 1 or 2% to about 5, 10 or 15% supplemental fiber, from about 50 or 60% to about 80, 85 or 90% moisture, all by weight of the pet food composition. A wet pet food composition as disclosed herein comprises, on a dry matter basis, a minimum protein level of about 9.5% to about 22%, a minimum fat level of about 8% to about 13%, a moisture level of about 65% to about 80%, a minimum supplemental fiber level of about 0.1% to about 3%, all by weight of the pet food composition.

A pet food composition as disclosed herein comprises, on a dry matter basis, from about 5, 10 or 20% to about 30, 40 or 50% of animal-derived ingredients, by weight of the pet food composition. Non-limiting examples of animal-derived ingredients include chicken, beef, pork, lamb, turkey (or other animal) protein or fat, egg, fishmeal and combinations thereof.

Where the pet food composition is in the form of a gravy, the pet food composition comprises at least 10% of a broth, or stock, non-limiting examples of which include vegetable beef, chicken or ham stock. Typical gravy compositions may comprise on a dry matter basis, from about 0.5% to about 5% crude protein, and from about 2% to about 5% crude fat.

When the pet food composition is in the form of biscuits, chews and other treats, the pet food composition comprises, on a dry matter basis, from about 20 or 22% to about 40 or 60% crude protein, by weight of the pet food composition. In an embodiment, the pet food composition comprises, on a dry matter basis, from about 5 or 10% to about 30 or 35% fat, by weight of the pet food composition.

The pet food composition can further comprise a wide range of other optional ingredients. Non-limiting examples of additional components include animal protein, plant protein, farinaceous matter, vegetables, fruit, egg-based materials, undenatured proteins, food grade polymeric adhesives, gels, polyols, starches, gums, flavorants, seasonings, salts, colorants, time-release compounds, minerals, vitamins, antioxidants, prebiotics, probiotics, aroma modifiers, textured wheat protein, textured soy protein, textured lupin protein, textured vegetable protein, breading, comminuted meat, flour, comminuted pasta, water, and combinations thereof.

Non-limiting examples of optional ingredients can include at least one vegetable. Non-limiting examples of vegetables include carrots, peas, potatoes, cabbage, celery, beans, corn, tomatoes, broccoli, cauliflower, leeks and combinations thereof.

Also useful herein, as an optional ingredient, is a filler. The filler can be a solid, a liquid or packed air. The filler can be reversible (for example thermo-reversible including gelatin) and/or irreversible (for example thermo-irreversible including egg white). Non-limiting examples of the filler include gravy, gel, jelly, aspic, sauce, water, air (for example including nitrogen, carbon dioxide, and atmospheric air), broth, and combinations thereof.

Non-limiting examples of colorants include, but are not limited to, synthetic or natural colorants, and any combination thereof. When present the colorants are from about 0.0001 %, 0.001%, or 0.005% to about 0.1%, 1% or 5% on a dry matter basis, of said pet food composition.

Additionally, probiotic microorganisms, such as *Lactobacillus* or *Bifidobacterium* species, for example, may be added to the pet food composition.

Also useful herein, as an optional ingredient, is at least one fruit. Non-limiting examples include tomatoes, apples, avocado, pears, peaches, cherries, apricots, plums, grapes, oranges, grapefruit, lemons, limes, cranberries, raspberries, blueberries, watermelon, cantaloupe, mushmellon, honeydew melon, strawberries, banana, and combinations thereof.

The pet food composition may contain other active agents such as long chain fatty acids and zinc. Suitable long chain fatty acids include alpha-linoleic acid, gamma linolenic acid, linoleic acid, eicosapentanoic acid, and docosahexanoic acid. Fish oils are a suitable source of eicosapentanoic acids (EPA) and docosahexanoic acid (DHA). The DHA level is at least about 0.05%, 0.1%, or 0.15% of the pet food composition, all on a dry matter basis. The EPA level is at least about 0.05%, 0.1% or 0.15% of the pet food composition, all on a dry matter basis.

The pet food composition may further comprise a source of carbohydrate. Grains or cereals such as rice, corn, milo, sorghum, barley, wheat, and the like are illustrative sources.

The pet food composition may also contain other materials such as dried whey and other dairy by products.

The pet food composition can be manufactured according to any known or otherwise effective technique suitable for making and formulating the desired pet food composition. The pet food composition can be manufactured by providing at least one protein ingredient, providing at least one fat ingredient, providing at least one antioxidant component and combining the protein, fat and antioxidant component.

The antioxidant component disclosed herein may be combined with an emulsifier. The combination of the emulsifier and the antioxidant component may improve the stability of the pet food composition ingredient. Non-limiting examples of emulsifiers include the following: choline salts, tannic acid, lecithin, sodium citrates, sodium phosphates, potassium phosphates, calcium phosphates, propylene glycol alginate, sorbitol, polyethylene, polysorbate 80, polysorbate 60, ammonium salts of phosphatidic acid, sucrose acetate isobutyrate, glycerol esters of wood rosins, potassium pyrophosphate, potassium polymetaphosphate, methyl cellulose, hydroxypropyl cellulose, hydroxypropyl methylcellulose, methyl ethyl cellulose, aluminum, calcium, sodium, magnesium, potassium and ammonium salts of fatty acids, mono- and diglycerides of fatty acids, acetic and fatty acid esters of glycerol, lactic and fatty acid esters of glycerol, citric and fatty acid esters of glycerol, diacetyltartaric and fatty acid esters of glycerol, mixed tartaric acetic and fatty acid esters of glycerol, sucrose esters of fatty acids, polyglycerol esters of fatty acids, polyglycerol esters of interesterified ricinoleic acid, propylene glycol mono- and di-esters, dioctyl sodium sulphosuccinate, sodium lactylate, calcium lactylate, sorbitan monostearate, sorbitan tristearate, sodium aluminum phosphate, bone phosphate, polydimethylsiloxane, maltitol and maltitol syrup, and combinations thereof. The emulsifier can be combined with the antioxidant component and then added to the pet food composition ingredient to be preserved which is then incorporated into the pet food composition. A sonicator can be used to form an emulsion of an antioxidant component and an emulsifier so that the emulsion can be incorporated into the pet food composition ingredient to be preserved which is then incorporated into the pet food composition.

The antioxidant component disclosed herein can be combined with a chelating agent.

The combination of the chelating agent and the antioxidant component may improve the stability of the pet food composition ingredient. Non-limiting examples of a chelating agent include the following: citric acid, ascorbic acid, Vitamin-B 12, chlorophyll, tannic acid, malic acid, sorbitol, gluconic acid, gluconate, and combinations thereof. The chelating agent can be combined with the antioxidant component and then added to the pet food composition ingredient to be preserved which is then incorporated into the pet food composition.

The antioxidant component is added to a pet food composition ingredient to increase the oxidative stability of the pet food composition ingredient in the pet food composition. For example, the waste stream was added to poultry fat, a common ingredient in pet food compositions. This addition of the waste stream increased the oxidative stability of that fat, as shown in Table K. The waste stream was added to the poultry fat at 2.5%, 5%, 10%, and 12.5% of the fat. Oxidative stability of the fat was tested in an Omnion OSI (oxidative stability index) instrument at 108 °C. The OSI instrument accelerates the development of oxidative rancidity so that the useful life of an oil, or an oil-containing material, can be determined and compared to other materials. At even the lowest level (2.5%), the waste stream increased the oxidative stability of the poultry fat almost five-fold in the OSI test. The waste stream preserved the poultry fat against oxidation, similar to Naturox® brand antioxidant, which is a standard commercial antioxidant preparation used in pet food. The waste stream was more effective at preserving the poultry fat than the purified chlorogenic acid standard, when adjusted for actual chlorogenic acid concentration in the waste stream, as shown in Table L. Other antioxidants and polyphenols in the waste stream also contribute to its preservation effectiveness.

**Table K. Preservation of Poultry Fat by Waste Stream**

| AOX Added to Poultry Fat* | OSI (hours)* |
|---|---|
| None | 3.4 |
| Naturox®* (0.3%) | 18.3 |
| CGA Standard* (0.09%) | 12.3 |
| Waste Stream (2.5%) | 15.5 |
| Waste Stream (5%) | 21.3 |
| Waste Stream (10%) | 22.4 |
| Waste Stream (12.5%) | 27.3 |

| | |
|---|---|
| * Poultry fat = chicken fat (Peacock™ from George Pfau & Sons Co., Jeffersonville, IN). OSI = Oxidative Stability Index. Naturox® = a commercial antioxidant commonly used in pet food (a mixture of vegetable oil, natural tocopherols, citric acid, and rosemary extract, from Kemin Industries, Ames, IA). CGA Standard chlorogenic acid, C3878, from Sigma-Aldrich Chemical Co., St. Louis, MO, CAS # 327-97-9, >95% pure. Waste Stream = sample A. | |

**Table L. Poultry Fat Preservation Based on CGA Content**

| AOX Added to Poultry Fat | CGA | OSI (hours) |
|---|---|---|
| None | 0.0 | 3.4 |
| Waste Stream (2.5%) | 0.063% | 15.5 |
| CGA Standard (0.09%) | 0.090% | 12.3 |
| Waste Stream (5%) | 0.124% | 21.3 |

See Table K above for details.

### Methods

The ORAC method is the most widely used and accepted measurement of antioxidant activity of foods and beverages. The ORAC values of numerous foods and beverages have been compiled into databases on antioxidant activity (see for example Wu et al., 2004, Lipophilic and hydrophilic antioxidant capacities of common foods in the United States, J. Agric. Food Chem., 52: 4026-37). ORAC is based on the oxidation of a fluorescent dye (fluorescein) by an oxidant [AAPH: 2,2'-azobis(2-amidino-propane) dihydrochloride] that can be monitored with a fluorescent plate reader. The addition of an antioxidant material (such as the waste stream described herein) slows down the oxidation of the fluorescein. The effectiveness of the antioxidant material is then quantitated by comparison to a standard antioxidant material [Trolox: 6-hydroxy-2,5,7,8-tetra-methylchroman-2-carboxylic acid].

ORAC measurements were done with a BMG Labtech Fluostar Optima fluorescent plate reader (Durham, NC) with injectors and Optima software (version 2.10 R2). The ORAC measurements were done at 37° C, and 96 well black flat bottom assay plates were used. Procedures were largely followed as previously described by Ou et al. (2001, Development and validation of an improved oxygen radical absorbance capacity assay using fluorescein as the fluorescent probe, J. Agric. Food Chem., 49: 4619-26) and Prior et al. (2003, Assays for hydrophilic and lipophilic antioxidant capacity [oxygen radical absorbance capacity (ORACFL) of plasma and other biological and food samples, J. Agric. Food Chem., 51: 3273-79].

Chlorogenic acids (CGAs) and caffeine were quantified by high performance liquid chromatography (HPLC) with UV detection (324 nm for chlorogenic acids; 280 nm for caffeine). A Waters Alliance HPLC system was used, with an Atlantis C18 column, and gradient elution 16 min gradient) using a mixture of 2% acetic acid (AcOH/water) and acetonitrile (ACN) beginning with 90% AcOH/water and 10% ACN. From application of 5-CQA and caffeine standard calibration curves, concentrations of chlorogenic acids and caffeine were calculated and quantified via known absorbance obtained with chlorogenic acids at 324 nm (UH Engelhardt et al., 1996, Determination of chlorogenic acids with lactones in roasted coffee, J. Sci. Food Agric. 71: 392-8) and caffeine at 280 nm. Individual CGAs measured included: 3-CQA (caffeoylquinic acid), 4-CQA, 5-CQA, 4-FQA (feruloylquinic acid), 5-FQA, 3,4-diCQA (dicaffeoylquinic acid), 3,5-diCQA, and 4,5-diCQA

The total amount of polyphenolic compounds was measured using the Folin method (see, for example, VA Singleton et al., 1999, Methods Enzymol. 299:152). The method is based on the formation of a blue color from the reaction of polyphenols with the Folin-Ciocalteu reagent, which is measured with a spectrophotometer. Results are expressed as equivalents of a standard material, from a standard curve. For the Folin analysis, a 600 mg/L solution of Gallic Acid (Acros Organics, Geel, Belgium) was used as the standard. An aliquot of standard, blank, or sample (up to 1 ml) was added to 5 ml deionized water, 1 ml of Folin-Ciocalteu reagent (VWR International, Inc., West Chester, PA) was added. After 5-8 min, 10 ml of 7% w/v sodium carbonate was added, and the total volume made up to 25 ml. After 2 hour, absorbance was measured at 760 nm.

The percent solids was measured by evaporation to dryness (minimum 12 hours at 60 C in forced air oven), or by refractive index. The Refractive Index (RI) was converted to percent solids by using a correlation established between RI and solids as determined by evaporation to dryness. Refractive Index was measured with a Bellingham & Stanley RFM 340 Refractometer (Norcross, GA) at 29 C.

Antioxidant Response Element (ARE): An antioxidant response element (ARE) reporter cell line (ARE32) was obtained from CXR Biosciences, Dundee, Scotland. The ARE32 cells were maintained in 'D-10' media which consisted of Dulbecco's Modified Eagle Medium (DMEM; catalog #11054, Invitrogen Co., Grand Island, NY) containing 10% Fetal Bovine Serum heat inactivated (FBS; catalog #10082-147, Invitrogen Co.), 2 mM Glutamax (catalog #35050, Invitrogen Co.), and 0.8 mg/ml Genetin G418 sulphate (G418; catalog #10131-027, Invitrogen Co.). Cells were subcultured every 3-4 days. The ARE assay was conducted as follows:
1. In a 96 well plate (Costar catalog #3610; Corning Co., Corning, NY), each well was seeded with 1x10⁴ cells in 100 ul D-10 media.
2. Cells were incubated at 37 C for 24 hours in a 5% CO₂ incubator.
3. The medium was replaced with 100 ul fresh D-10. Cells were treated with test material in dose response starting at 10%, then 1:2 dilutions for 8 wells total in duplicate. A control (no test material) and a positive control (10 uM TBHQ; tert-butylhydroquinone, Aldrich Co., St. Louis, MO) were included.
4. After treatment, 100 ul D-10 media was added, for a final assay volume of 200 ul.
5. Cells were incubated at 37 C for 24 hours in a 5% CO₂ incubator.
6. Media (100 ul) was removed and Steady Glo Assay System (100 ul; catalog #E2510, Promega Co., Madison, WI) was added.
7. After shaking for 10 minutes, the assay was read using an EnVision 2101 Multilabel

Reader (Perkin Elmer Co., Turku, Finland), using the Promega luciferase protocol. Results were calculated as the percent increase in ARE response versus the control.

Oxygen Stability Index (OSI). Oxidative stability was determined in an Oxidative Stability Instrument (OSI, Omnion, Inc., Rockland, MA) at 108 C. A Ross high-shear mixer was used to thoroughly mix the poultry fat and antioxidant together before testing in the OSI instrument. Five grams (5 g) of test material was placed in a 50 ml test tube and air was passed through the test chamber at 5.1 psi. The Oil Stability Index (OSI) is the time, in hours, until the sample begins to rapidly oxidize.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

The citation of any document is not an admission that it is prior art with respect to any invention disclosed or claimed herein or that it alone, or in any combination with any other reference or references, teaches, suggests or discloses any such invention.

While particular embodiments of the present invention have been illustrated and described, it would be obvious to those skilled in the art that various other changes and modifications can be made without departing from the scope of the invention. It is therefore intended to cover in the appended claims all such changes and modifications that are within the scope of this invention.

## Claims

1. Use of an antioxidant component derived from a decaffeination stream produced during the decaffeination process of coffee beans to preserve at least one ingredient in a moist pet food composition, and/or wet pet food composition, and/or dry pet food composition, wherein the antioxidant component comprises chlorogenic acid.

2. The use of claim 1, wherein said chlorogenic acid comprises Di-CQAs and Mono-CQAs.

3. The use of claim 2, wherein said Di-CQAs are enriched.

4. The use of claim 2, wherein said Di-CQAs are present in an amount greater than Mono-CQAs.

5. The use of claim 2, wherein a ratio of Di-CQAs to Mono-CQAs is from about 3:1 to about 1.3:1.

6. The use of claim 1, wherein said pet food composition is complete and nutritionally balanced.

## Patentansprüche

1. Verwendung einer Antioxidanskomponente, die von einem Entcoffeinierungsstrom abgeleitet ist, der während des Entcoffeinierungsverfahrens von Kaffeebohnen hergestellt wird, zum Konservieren mindestens eines Inhaltsstoffs in einer feuchten Haustierfutterzusammensetzung und/oder nassen Haustierfutterzusammensetzung und/oder trockenen Haustierfutterzusammensetzung, wobei die Antioxidanskomponente Chlorogensäure umfasst.

2. Verwendung nach Anspruch 1, wobei die Chlorogensäure Di-CQAs und Mono-CQAs umfasst.

3. Verwendung nach Anspruch 2, wobei die Di-CQAs angereichert sind.

4. Verwendung nach Anspruch 2, wobei die Di-CQAs in einer größeren Menge als Mono-CQAs vorhanden sind.

5. Verwendung nach Anspruch 2, wobei ein Verhältnis von Di-CQAs zu Mono-CQAs von etwa 3:1 bis etwa 1,3:1 beträgt.

6. Verwendung nach Anspruch 1, wobei die Haustierfutterzusammensetzung vollständig und ernährungsphysiologisch ausgewogen ist.

## Revendications

1. Utilisation d'un composant antioxydant dérivé d'un courant de décaféination produit pendant le processus de décaféination de grains de café pour préserver au moins un ingrédient dans une composition alimentaire humide pour animaux de compagnie, et/ou une composition alimentaire mouillée pour animaux de compagnie, et/ou une composition alimentaire sèche pour animaux de compagnie, dans laquelle le composant antioxydant comprend un acide chlorogénique.

2. Utilisation selon la revendication 1, dans laquelle ledit acide chlorogénique comprend des Di-CQA et des Mono-CQA.

3. Utilisation selon la revendication 2, dans laquelle lesdits Di-CQA sont enrichis.

4. Utilisation selon la revendication 2, dans laquelle lesdits Di-CQA sont présents en une quantité supérieure aux Mono-CQA.

5. Utilisation selon la revendication 2, dans laquelle un rapport des Di-CQA aux Mono-CQA va d'environ 3:1 à environ 1,3:1.

6. Utilisation selon la revendication 1, dans laquelle ladite composition alimentaire pour animaux de compagnie est complète et nutritionnellement équilibrée.
